# EUROPÄISCHE PATENTSCHRIFT

(11) **EP 0 966 453 B1**
(45) Veröffentlichungstag und Bekanntmachung des Hinweises auf die Patenterteilung: **23.07.2003**
(21) Anmeldenummer: 98912327.8
(22) Anmeldetag: 18.02.1998
(51) Int. Cl.: C07D 275/06, A01N 43/80

(54) **SACCHARIN-5-CARBONYL-CYCLOHEXAN-1,3,5-TRIONDERIVATE, DEREN HERSTELLUNG UND DEREN VERWENDUNG ALS HERBIZIDE**
SACCHARINE-5-CARBONYL-CYCLOHEXANE-1,3,5-TRIONE DERIVATIVES, THEIR PREPARATION AND THEIR USE AS HERBICIDES
DERIVES DE SACCHARINE-5-CARBONYL-CYCLOHEXANE-1,3,5-TRIONE, LEUR FABRICATION ET LEUR UTILISATION COMME HERBICIDES

(30) Priorität: 10.03.1997 DE 19709697
(43) Veröffentlichungstag der Anmeldung: 29.12.1999
(73) Patentinhaber: BASF AKTIENGESELLSCHAFT, 67056 Ludwigshafen (DE)
(72) Erfinder: WALTER, Helmut, D-67283 Obrigheim (DE); PLATH, Peter, D-67227 Frankenthal (DE); KARDORFF, Uwe, D-68159 Mannheim (DE); WITSCHEL, Matthias, D-67061 Ludwigshafen (DE); HILL, Regina, Luise, D-67346 Speyer (DE); VON DEYN, Wolfgang, D-67435 Neustadt (DE); ENGEL, Stefan, D-65510 Idstein (DE); OTTEN, Martina, D-67069 Ludwigshafen (DE); MISSLITZ, Ulf, D-67433 Neustadt (DE); WESTPHALEN, Karl-Otto, D-67346 Speyer (DE)
(86) Internationale Anmeldenummer: EP9800926
(87) Internationale Veröffentlichungsnummer: WO98040366

(56) Entgegenhaltungen:
- EP-A- 0 283 261
- WO-A-96/05182
- WO-A-97/08164
- DE-A- 19 532 311

## Beschreibung

Gegenstand der vorliegenden Anmeldung sind Saccharin-5-carbonylcyclohexan-1,3,5-trionderivate der Formel I 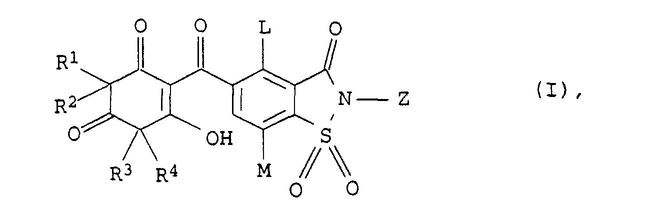 in der die Substituenten folgende Bedeutung haben:
- L: C₁-C₃-Alkyl,
- Z: C₁-C₄-Alkyl,
- M: Wasserstoff, C₁-C₃-Alkyl,
- R¹, R², R³, R⁴: C₁-C₄-Alkyl;
sowie landwirtschaftlich brauchbare Salze der Verbindung I.

Gegenstand der Erfindung sind ferner herbizide Mittel, enthaltend die Verbindungen I sowie Verfahren zur Bekämpfung unerwünschten Pflanzenwuches mit den Saccharinderivaten I.

Aus der WO 96/05182 sind herbizid wirksame Saccharincarbonyl-cyclohexandionderivate bekannt.

Die EP-A 252 298 beschreibt herbizide Benzoyl-1,3,5-cyclohexantrione, die allerdings keine Saccharin-Struktur aufweisen.

Bekannt ist ferner die Verwendung von Saccharinderivaten als Fungizide, z.B. JP-Publikation 72/00419 und 73/35457 und in der Pharmazie, z.B. EP-A 594 257.

WO 97/08164 und DE 195 32311 beschreiben Cyclohexan dion-derivate, EP 0 283 261 Cyclohexantrione derivate.

Die herbiziden Eigenschaften der bekannten Verbindungen sowie die Verträglichkeit gegenüber Kulturpflanzen können jedoch nur bedingt befriedigen.

Die Aufgabe bestand darin, neue Saccharinderivate mit verbesserten Eigenschaften zu finden.

Demgemäß wurden die eingangs definierten Saccharin-5-carbonyl-cy clohexan-1,3,5-trionderivate der allgemeinen Formel I gefunden.

Verbindungen der Formel I erhält man gemäß Schema 1, indem man Cyclohexan-1,3,5-trione der Formel II mit einem Saccharin-5-carbonsäurechlorid der Formel III acyliert und den erhaltenen Enolester der Formel IV in Gegenwart eines Katalysators zum Wirkstoff der Formel I umlagert.

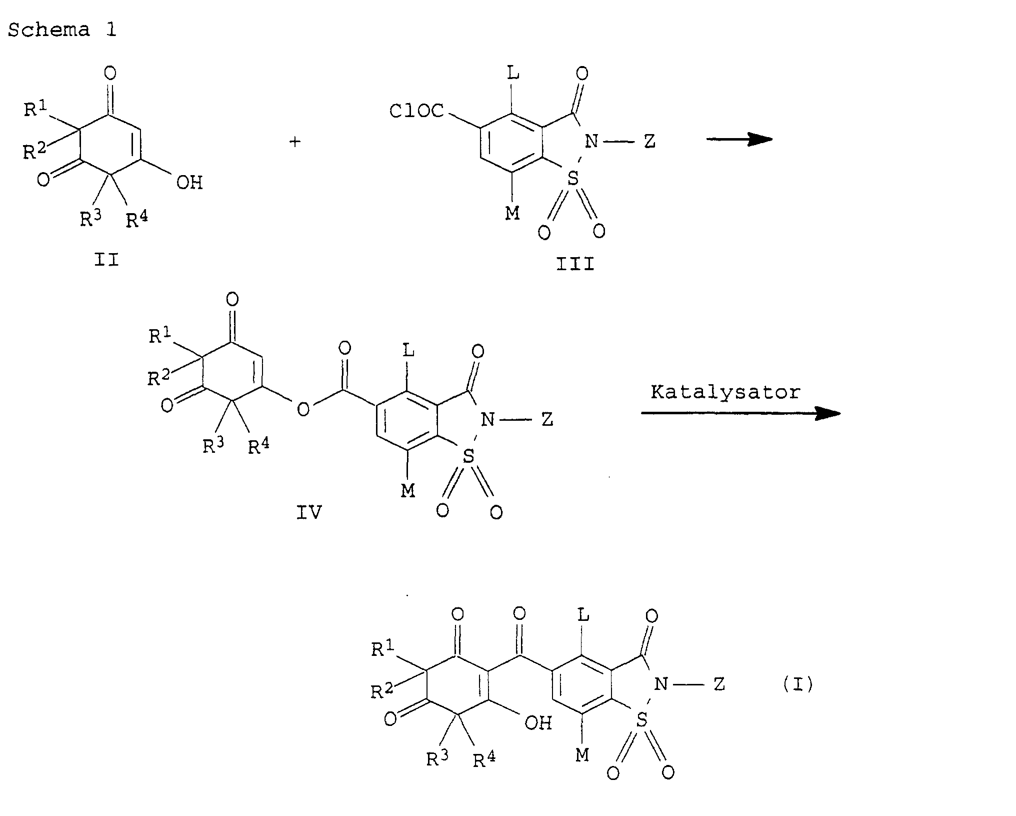

In dem obigen Schema 1 haben die Substituenten R¹ bis R⁴, L, M und Z die eingangs angegebene Bedeutung.

Der erste Schritt der Reaktionsfolge in Schema 1 erfolgt durch Zugabe des Säurechlorids III zur Lösung oder Suspension des Cyclohexan-1,3,5-trions II in Gegenwart einer Hilfsbase. Die Reaktanden und die Hilfsbase setzt man bevorzugt in äquimolaren Mengen ein, jedoch kann ein geringer Überschuß von 1,2 bis zu 1,5 Moläquivalenten der Hilfsbase von Vorteil sein. Als Lösungsmittel können Methylenchlorid, Tetrahydrofuran, Essigsäureethylester, Toluol oder bevorzugt Acetonitril verwendet werden. Als Hilfsbase eignen sich Alkalicarbonate, Pyridin oder tertiäre Alkylamine, bevorzugt Triethylamin. Während der Zugabe des Säurechlorids wird die Reaktionsmischung vorzugsweise auf 0 bis 10°C gekühlt, danach wird bei einer Temperatur von 20 bis 70°C , insbesondere 25 bis 40°C gerührt, bis die Umsetzung beendet ist.

Der Enolester IV kann vor der Umlagerung isoliert werden, jedoch wird die Reaktion bevorzugt in der Weise durchgeführt, daß man dem Reaktionsgemisch zwei bis vier, bevorzugt 2,5 Äquivalente Triethylamin zusetzt und anschließend bei 25°C 2 bis 10, insbesondere 3 Mol-% einer Cyanoverbindung wie Acetoncyanhydrin oder bevorzugt Trimethylsilylcyanid zufügt und anschließend bei einer Temperatur von 20 bis 40°C, bevorzugt bei 25°C rühren läßt, bis der Enolester IV nicht mehr vorhanden ist. Beispiele zur cyanidkatalysierten Umlagerung von Enolestern von Cyclohexan-1,3,5-trionen findet man in EP-A 252 298.

Die Aufarbeitung erfolgt, indem das Reaktionsgemisch mit 5 %iger Salzsäure oder Schwefelsäure angesäuert wird und dann mit einem Lösungsmittel wie Essigsäureethylester oder Methylenchlorid extrahiert wird. Nach Trocknen des Extraktes über Natriumsulfat ) oder Magnesiumsulfat wird das Lösungsmittel im Vakuum abdestilliert und das Rohprodukt, falls erforderlich, einer Reinigung unterworfen. Zur Reinigung kann das Reaktionsprodukt beispielsweise chromatographiert (Kieselgel, Cyclohexan/Essigsäureethylester) oder umkristallisiert werden (Methanol/Wasser oder Eisessig/Wasser). Eine weitere Reinigungsmethode ist die Extraktion einer Essigsäureethylesterlösung des Rohproduktes mit einer wässrigen Alkalicarbonatlösung, wobei das Endprodukt in die wässrige Phase übergeht. Ansäuern der wässrigen Lösung und erneute Extraktion liefert nach Trocknung und Entfernen des Lösungsmittels das Endprodukt in reinerer Form.

Die als Ausgangsmaterial verwendeten Cyclohexan-1,3,5-trione der Formel II sind bekannt und können in an sich bekannter Weise hergestellt werden [vgl. Spitzer, Monatshefte der Chemie 11, 104 (1890). Riedl et al., Liebigs Ann. Chem. 585, 209 (1954) und Murin et al. Chem. Ber. 92, 2033 (1959)]. Ein Verfahren zur Herstellung des besonders bevorzugten 2,2,4,4-Tetramethyl-cyclohexan-1,3,5-trions ist in der EP-A 283 152 beschrieben.

Die als Ausgangsmaterial verwendeten Säurechloride der Formel III sind ebenfalls bekannt. Sie werden durch Umsetzung einer geeignet substituierten Saccharin-5-carbonsäure mit Thionylchlorid erhalten. Die Synthese von substituierten Saccharin-5-carbonsäuren ist z.B. in der DE 44 27 996 beschrieben.

Bei der eingangs angegebenen Definitionen der Verbindungen I wurden Sammelbegriffe verwendet, die allgemein repräsentativ für die folgenden Gruppen stehen:
**Alkyl:** geradkettige oder verzweigte Alkylgruppen mit 1 bis 4 Kohlenstoffatomen, z.B. C₁-C₄-Alkyl wie Methyl, Ethyl, Propyl, 1-Methylethyl, Butyl, 1-Methyl-propyl, 2-Methylpropyl und 1,1-Dimethylethyl;
**Alkoxy:** geradkettige oder verzweigte Alkylgruppen mit 1 bis 3 Kohlenstoffatomen wie vorstehend genannt, welche über ein Sauerstoffatom (-O-) an das Gerüst gebunden sind, z.B. C₁-C₃-Alkoxy wie Methyloxy, Ethyloxy, Propyloxy und 1-Methylethyloxy;
**Cycloalkyl:** monocyclische Alkylgruppen mit 3 bis 8 Kohlenstoffringgliedern, z.B. Cyclopropyl, Cyclobutyl, Cyclopentyl, Cyclohexyl, Cycloheptyl und Cyclooctyl;
**Alkenyl:** geradkettige oder verzweigte Alkenylgruppen mit 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-l-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, l-Ethyl-2-methyl-1-propenyl und 1-Ethyl-2-methyl-2-propenyl;
**Alkinyl:** geradkettige oder verzweigte Alkinylgruppen mit 3 bis 5 Kohlenstoffatomen und einer Dreifachbindung in einer beliebigen Position, z.B. C₃-C₅-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl und 1-Ethyl-2-propinyl;
**Halogen:** Fluor, Chlor, Brom und Jod.

Im Hinblick auf die bestimmungsgemäße Verwendung als Herbizide sind Saccharin-5-carbonyl-cyclohexan-1,3,5-trionderivate der Formel I bevorzugt, in der die Substituenten die folgende Bedeutung haben:
- L: Methyl, Ethyl, besonders bevorzugt Methyl;
- Z: C₁-C₄-Alkyl, weiterhin bevorzugt Methyl, Ethyl, i-Propyl, i-Butyl, t-Butyl, besonders bevorzugt Methyl und Ethyl und ganz besonders bevorzugt Methyl;
- M: Wasserstoff, Methyl, Ethyl; weiterhin bevorzugt Wasserstoff, Methyl, Ethyl; besonders bevorzugt Wasserstoff und Methyl
- R¹, R², R³, R⁴: Methyl, Ethyl, n-Propyl, n-Butyl; weiterhin bevorzugt Methyl und Ethyl; besonders bevorzugt Methyl.

Bevorzugt sind auch die Kombinationen der obengenannten bevorzugten Substituenten.

Ganz besonders bevorzugt sind Saccharinderivate der Formel I, in der die Substituenten
- L und R¹ bis R⁴: für Methyl,
- Z: für Methyl und
- M: für Wasserstoffe oder Methyl stehen.

Die Verbindungen I können in Form ihrer landwirtschaftlich brauchbaren Salze vorliegen, wobei es auf die Art des Salzes im allgemeinen nicht ankommt. Üblicherweise werden die Salze von solchen Basen in Betracht kommen, welche die herbizide Wirkung von I nicht negativ beeinträchtigen.

Als basische Salze eignen sich besonders diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze sowie die Ammoniumsalze, die ein bis drei C₁-C₄-Alkyl, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium-, und Trimethyl-(2-hydroxyethyl)-ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri-(C₁-C₄-)alkylsulfoxoniumsalze.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Sektrum) eingesetzt. Die erfindungsgemäßen Verbindungen I können beispielsweise wie folgt formuliert werden:
I 20 Gewichtsteile der Verbindung Nr. 1.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II 20 Gewichtsteile der Verbindung Nr. 1.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
III 20 Gewichtsteile des Wirkstoffs Nr. 1.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
IV 20 Gewichtsteile des Wirkstoffs Nr. 1.1 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
V 3 Gewichtsteile des Wirkstoffs Nr. 1.1 werden mit 97 Gewichtsteilen feinteiligem Kaolin vermischt. Man erhält auf diese Weise ein Stäubemittel, das 3 Gew. % des Wirkstoffs enthält.
VI 20 Gewichtsteile des Wirkstoffs Nr. 1.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII 1 Gewichtsteil der Verbindung 1.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII 1 Gewichtsteil der Verbindung 1.1 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole,
1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

) Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Herstellungsbeispiele

Wirkstoff aus 2,2,4,4-Tetramethyl-cyclohexan-1,3,5-trion und 2,4-Dimethylsaccharin-5-carbonsäurechlorid (Nr. 1 in Tabelle 1)

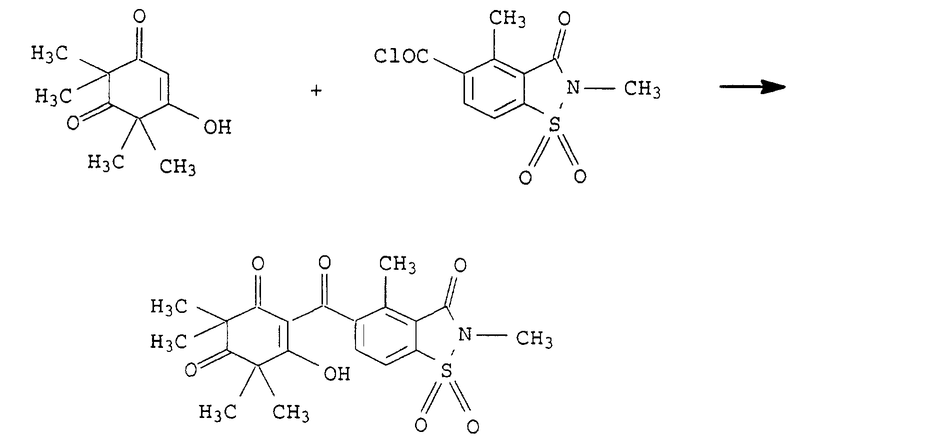

Man suspendiert 0,91 g (0,005 Mol) 2,2,4,4-Tetramethyl-cyclohexan-1,3,5-trion in 25 ml Acetonitril und fügt 0,6 g (0,005 Mol) Triethylamin zu. Anschließend gibt man bei 25°C auf einmal 1,37 g (0,005 Mol) 2,4-Dimethylsaccharin-5-carbonsäurechlorid zu und läßt die Reaktionsmischung ohne Kühlung 4 Std. rühren. Danach setzt man erneut 1,2 g (0,012 Mol) Triethylamin und anschließend 10 Tropfen Trimethylsilylcyanid zu und läßt 16 Std. bei 25°C rühren. Zur Vervollständigung der Umlagerung des Enolesters wird noch 2 Std. auf 40°C erwärmt.

Zur Aufarbeitung wird das Reaktionsgemisch am Rotationsverdampfer zur Trockne eingeengt, der Rückstand mit 30 ml Wasser versetzt, mit 5 %iger HCl auf pH1 angesäuert und dreimal mit Essigsäureethylester extrahiert. Nach Trocknen des Extraktes über Natriumsulfat wird das Lösungsmittel am Rotationsverdampfer abgezogen, wobei 2,1 g eines Feststoffs erhalten werden. Nach Umkristallisation aus einer Mischung von 5 Teilen Eisessig und 1 Teil Wasser erhält man 0,9 g (43 % d.Th. Ausbeute) eines weißen Feststoffs mit Fp. 210°C.

In gleicher Weise können folgende Verbindungen in Tabelle 1 erhalten werden:

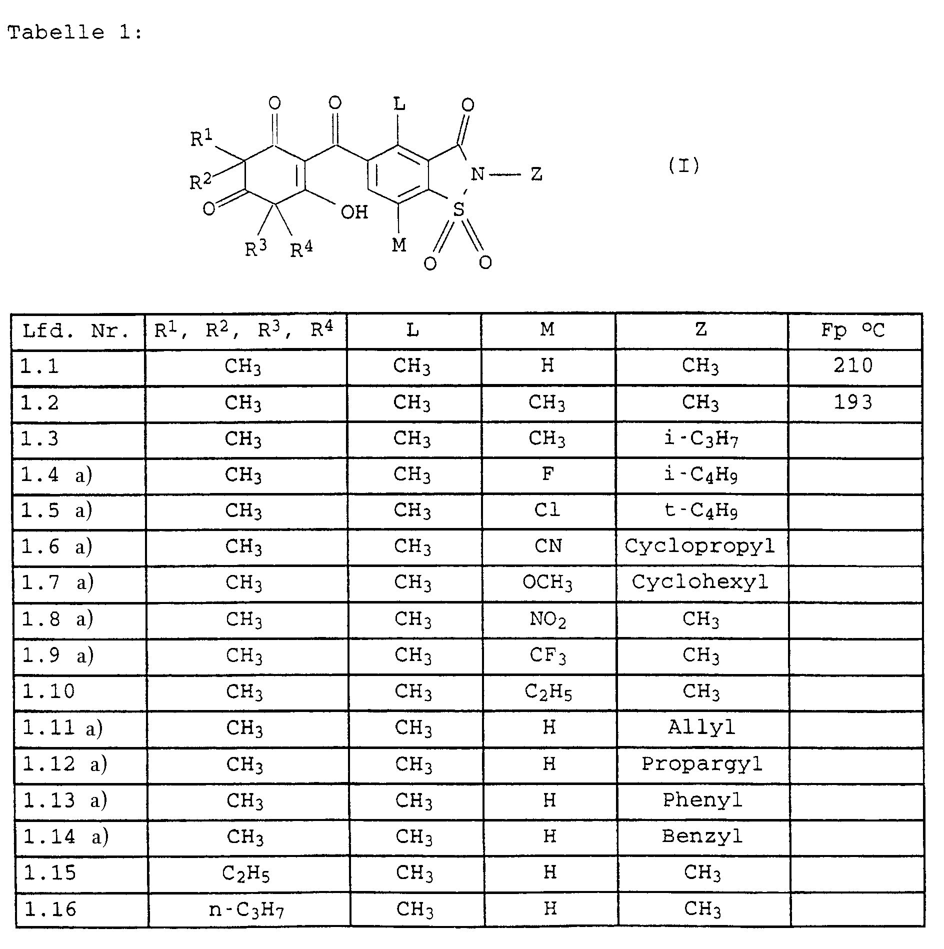

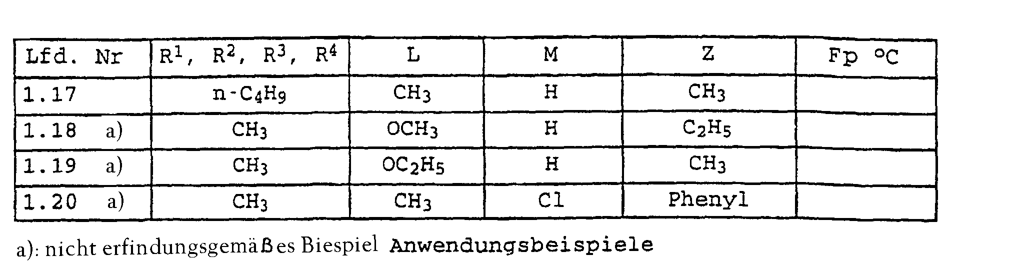

Die herbizide Wirkung der Verbindungen der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges keimen der Testpflanzen, sofern dies nicht durch die Wirkstoffe beeinträchtigt wurde. Die Aufwandmenge für die Vorauflaufbehandlung betrug 0.5 bzw. 0.25 kg/ha a. S.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf, die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und keine Schädigung oder normaler Wachstumsverlauf.

Die in den Gewächshausversuchen verwendeten Pflanzen setzten sich aus folgenden Arten zusammen:

| | | | |
|---|---|---|---|
| Lateinischer Name | Deutscher Name | Englischer Name | Abkürzung |
| Gossypium hirsutum | Baumwolle | cotton | GOSHI |
| Amaranthus retroflexus | Zurückgekrümmter Fuchsschwanz | redroot pigweed | AMARE |
| Digitaria sanguinalis | Blutfingerhirse | fingergrass | DIGSA |
| Echinochloa crus-galli | Hühnerhirse | barnyardgrass | ECHCG |
| Setaria faberii | Borstenhirse | giant foxtail | SETFA |
| Setaria viridis | Grüne Borstenhirse | green foxtail | SETVI |

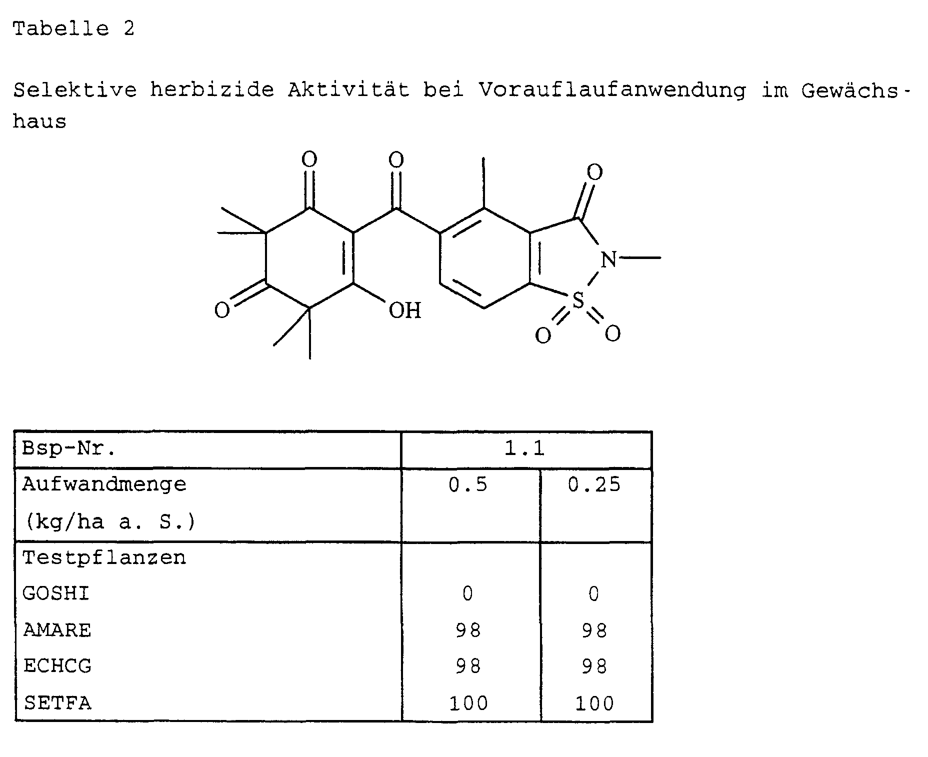

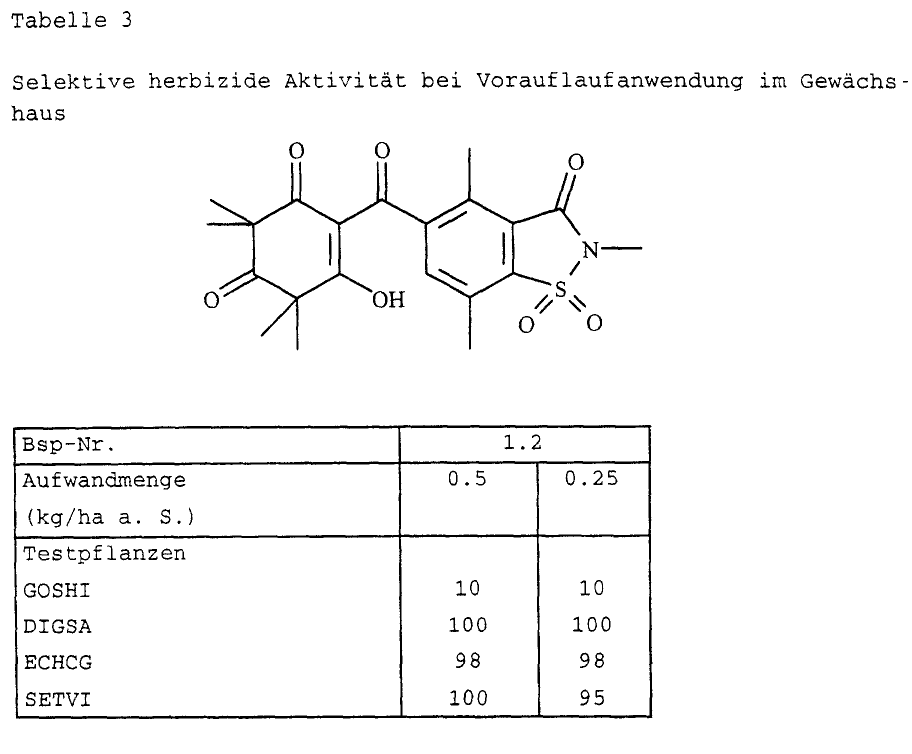

## Patentansprüche

1. Saccharin-5-carbonyl-cyclohexan-1,3,5-trionderivate der Formel I 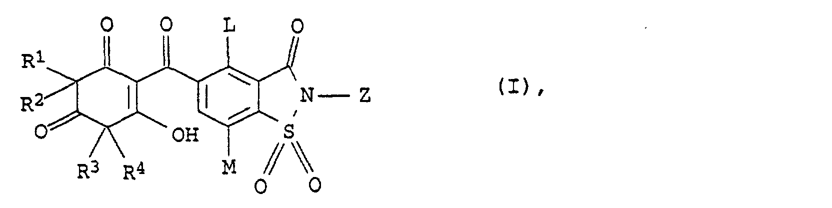 in der die Substituenten folgende Bedeutung haben:
L C₁-C₃-Alkyl;
Z C₁-C₄-Alkyl;
M Wasserstoff, C₁-C₃-Alkyl;
R¹, R², R³, R⁴ C₁-C₄-Alkyl;
sowie landwirtschaftlich brauchbare Salze der Verbindung I.

2. Saccharinderivate der Formel I nach Anspruch 1, in der L für Methyl- oder Ethyl steht.

3. Saccharinderivate der Formel I nach einem der Ansprüche 1 bis 2, in der Z für Methyl, Ethyl, i-Propyl, i-Butyl oder t-Butyl steht.

4. Saccharinderivate der Formel I nach einem der Ansprüche 1 bis 3, in der M für Wasserstoff, Methyl oder Ethyl steht.

5. Saccharinderivate der Formel I nach einem der Ansprüche 1 bis 4, in der R¹ bis R⁴ für Methyl, Ethyl, n-Propyl oder n-Butyl stehen.

6. Saccharinderivate der Formel I nach einem der Ansprüche 1 bis 5, in der R¹ bis R⁴ für Methyl oder Ethyl stehen.

7. Saccharinderivate der Formel I nach Anspruch 1, in der L und R¹ bis R⁴ für Methyl, Z für Methyl und M für Wasserstoff oder Methyl stehen.

8. Herbizides Mittel, enthaltend mindestens ein Saccharinderivate der Formel I gemäß Anspruch 1 und übliche inerte Zusatzstoffe.

## Claims

1. Saccharin-5-carbonylcyclohexane-1,3,5-trione derivatives of the formula I 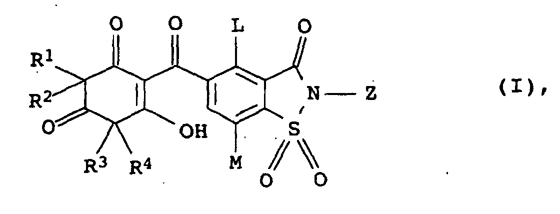 where:
L is C₁-C₃-alkyl;
Z is C₁-C₄-alkyl;
M is hydrogen, C₁-C₃-alkyl;
R¹, R², R³, R⁴ are each C₁-C₄-alkyl;
and agriculturally useful salts of compound I.

2. Saccharin derivatives of the formula I as claimed in claim 1, where L is methyl or ethyl.

3. Saccharin derivatives of the formula I as claimed in any of claims 1 to 2, where Z is methyl, ethyl, i-propyl, i-butyl or t-butyl.

4. Saccharin derivatives of the formula I as claimed in any of claims 1 to 3, where M is hydrogen, methyl or ethyl.

5. Saccharin derivatives of the formula I as claimed in any of claims 1 to 4, where R¹ to R⁴ are each methyl, ethyl, n-propyl or n-butyl.

6. Saccharin derivatives of the formula I as claimed in any of claims 1 to 5, where R¹ to R⁴ are each methyl or ethyl.

7. Saccharin derivatives of the formula I as claimed in claim 1, where L and R¹ to R⁴ are each methyl, Z is methyl and M is hydrogen or methyl.

8. A herbicidal composition comprising at least one saccharin derivative of the formula I as claimed in claim 1 and customary inert additives.

## Revendications

1. Dérivés de saccharine-5-carbonylcyclohexane-1,3,5-trione de formule I 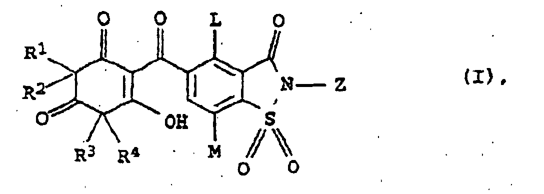 dans laquelle les substituants présentent la signification suivante:
L représente un groupe alkyle en C₁ à C₃,
Z représente un groupe alkyle en C₁ à C₄,
M représente un atome d'hydrogène, un groupe alkyle en C₁ à C₃,
R¹, R², R³ R⁴ représentent un groupe alkyle en C₁ à C₄,
ainsi que des sels du composé I Utilisables en agriculture.

2. Dérivés de saccharine de formule I selon la revendication 1, dans lesquels L représente un groupe méthyle ou un groupe éthyle.

3. Dérivés de saccharine de formule I selon l'une quelconque des revendications 1 à 2, dans lesquels Z représente un groupe méthyle, un groupe éthyle, un groupe i-propyle, un groupe i-butyle ou un groupe t-butyle.

4. Dérivés de saccharine de formule I selon l'une quelconque des revendications 1 à 3, dans lesquels M représente un atome d'hydrogène, un groupe méthyle ou un groupe éthyle.

5. Dérivés de saccharine de formule I selon l'une quelconque des revendications 1 à 4, dans lesquels R¹ à R⁴ représentent un groupe méthyle, un groupe éthyle, un groupe n-propyle ou un groupe n-butyle.

6. Dérivés de saccharine de formule I selon l'une quelconque des revendications 1 à 5 dans lesquels R¹ à R⁴ représentent un groupe méthyle ou un groupe éthyle.

7. Dérivés de saccharine de formule I selon la revendication 1, dans lesquels L et R¹ à R⁴ représentent un groupe méthyle, Z représente un groupe méthyle et M représente un atome d'hydrogène ou un groupe méthyle.

8. Agent herbicide, contenant au moins un dérivé de saccharine de formule I selon la revendication 1 et des additifs inertes usuels.
